# EUROPEAN PATENT SPECIFICATION

(11) **EP 2 983 700 B1**
(45) Date of publication and mention of the grant of the patent: **22.08.2018**
(21) Application number: 14716793.6
(22) Date of filing: 08.04.2014
(51) Int. Cl.: A61K 31/198, A61K 31/385, A61K 38/48, A61P 15/00

(54) **COMPOSITION FOR THE TREATMENT OF ENDOMETRIOSIS**
ZUSAMMENSETZUNG ZUR BEHANDLUNG VON ENDOMETRIOSE
COMPOSITION POUR LE TRAITEMENT DE L'ENDOMÉTRIOSE

(30) Priority: 09.04.2013 EP 13425052
(43) Date of publication of application: 17.02.2016
(73) Proprietor: Pizeta Group S.r.l., 06135 Ponte San Giovanni (PG) (IT)
(72) Inventor: DE SETA, Francesco, 34151 Trieste (IT); LEONARDI, Lucio, 06100 Perugia (IT)
(74) Representative: Pistolesi, Roberto
(86) International application number: PCT/EP2014/057054
(87) International publication number: WO 2014/166947

(56) References cited:
- EP-A1- 2 305 238
- US-A1- 2008 031 980
- US-A1- 2013 034 530
- DATABASE WPI Week 201334 Thomson Scientific, London, GB; AN 2013-E22172 XP002701178, & IT 1 398 281 B (DIFASS SA) 22 February 2013 (2013-02-22)
- PITTALUGA E ET AL: "More than antioxidant: N-acetyl-L-cysteine in a murine model of endometriosis", FERTILITY AND STERILITY, ELSEVIER SCIENCE INC, NEW YORK, NY, USA, vol. 94, no. 7, 1 December 2010 (2010-12-01), pages 2905-2908, XP027517760, ISSN: 0015-0282, DOI: 10.1016/J.FERTNSTERT.2010.06.038 [retrieved on 2010-07-23] cited in the application
- CLIFFORDA. SIMMONS: "THE RELIEF OF PAIN IN SPASMODIC DYSMENORRHOEA BY BROMELAIN", THE LANCET, vol. 272, no. 7051, 1 October 1958 (1958-10-01), pages 827-830, XP055070852, ISSN: 0140-6736, DOI: 10.1016/S0140-6736(58)90379-9

## Description

The present invention relates to a new composition, preferably with delayed release, containing N-acetyl cysteine, alpha-lipoic acid, and bromelain, in addition to the use thereof in the treatment of endometriosis, dysmenorrhoea and/or pelvic pain.

### STATE OF THE ART

Endometriosis is a chronic, complex disease, which originates from the abnormal presence of tissues that usually line the inner wall of the uterus, otherwise known as the endometrium, in other organs, such as the ovaries, fallopian tubes, peritoneum, vagina, or intestines.

This results in internal bleeding, acute and chronic inflammation, and cicatrisation, which could lead to adhesions and infertility.

The disease usually manifests with acute and/or chronic pain, which can reach such levels as to have a disabling effect. These extremely characteristic symptoms tend to appear in particular during the menstrual cycle or during the intermenstrual period and worsen upon evacuation or urination. It is precisely this pro-inflammatory state that is responsible for the dysmenorrhoea, pain, and infertility of patients affected by the disease.

A number of mediators, including cell adhesion molecules such as vascular cell adhesion molecule-1 (VCAM-1), pro-inflammatory cytokines such as tumour necrosis factor-α (TNF-α), interleukin-6 and IL-8, and chemokines such as monocyte chemotactic protein-1 (MCP-1), play key roles in the pathogenesis of endometriosis (Burney et al., 2012).

The pathogenic cascade of inflammation is brought about by the enzyme COX (cyclooxygenase), which, from arachidonic acid, leads to the formation of prostanoids, the main actors of inflammation. It has been highlighted how increased expression of the COX-2 enzyme in endometrial tissue causes the formation of PGE2 and PGF2 alpha prostaglandins as mediators of pain.

To date, no definitive endometriosis treatments have been identified yet. Until now, the management of this disease has concentrated on reducing symptoms or stopping the progression of the foci of endometriosis and has included medical and surgical treatments. The surgical treatment, carried out alone or in combination with medical treatment, is a common treatment for all stages of endometriosis. The aim of this treatment is to remove visible areas of endometriosis and restore the anatomy through lysis of adhesions. However, not everyone agrees that surgery alone gives greater relief from the symptoms of pain with respect to medical treatment. In fact, localisation, extent, and chronicity of the endometriotic process can lead to diverse clinical scenarios. The medical treatments currently available for endometriosis aim to reduce the synthesis of oestrogen, induce atrophy of the endometrial implants, interrupt the stimulation/bleeding cycle, and inhibit the formation/progression of new endometriotic foci.

Oral contraceptives, androgenic agents (such as danazol), progestin agents (such as medroxyprogesterone acetate), and gonadotropin releasing hormone agonists (GnRH-a - such as leuprolide acetate, goserelin acetate, nafarelin acetate) have been used so far as the main form of treatment for endometriosis. Among these, GnRH agonists represent the most widespread medical treatment for endometriosis, since they are capable of establishing a state of "pharmacological menopause" and therefore an important state of hypoestrogenism. Danazol, progestational agents, GnRH-a, and oral contraceptives display, in the short term, comparable success rates as regards relieving the symptoms of endometriosis, but their effectiveness remains only partial in terms of reducing the size of endometriosis-related lesions (Endometriosis : ancient disease, ancient treatments. Nezhat C, Nezhat F, Nezhat C. Fertil Steril. 2012 Dec;98(6 Suppl):S1-62. doi: 10.1016/j.fertnstert.2012.08.001. Epub 2012 Oct 17. Systematic review of therapies for noncyclic chronic pelvic pain in women. Yunker A, Sathe NA, Reynolds WS, Likis FE, Andrews J. Obstet Gynecol Surv. 2012 Jul;67(7):417-25. doi: 10.1097/OGX.0b013e31825cecb3. Review).

Not only do the short-term benefits of the conventional medical treatments involve side effects, they also do not offer a permanent solution to the risk of recurrence of the disease once the treatment is finished. The stigma of these side effects linked to conventional medicine has prompted several study groups to emphasize the role of nonconventional medicine. For example, although clinical trials on herbal formulas used in Chinese folk medicine (commonly prepared as both a boiled decoction and as dried extracts, or taken in pill form) have reported encouraging results as regards symptoms such as dysmenorrhoea, dysuria, dyschezia, and menorrhagia, such treatments have not been effective in permanently curing patients affected by the aforesaid disease. Therefore, there is a need to identify an alternative treatment, which can reduce the pain symptoms, without side effects, and which is well tolerated by the patient.

### BRIEF DESCRIPTION OF THE DRAWINGS

Figure 1. Analysis of the expression of VCAM-1 by human endometriotic endothelial cell (EEC), stimulated with TNFα.
Figure 2. Evaluation of IL-8 mRNA expression in endometriotic stromal cells (ESC) incubated with NAC, AL, Br alone or in admixture.
Figure 3. Evaluation of IL-6 mRNA expression in endometriotic stromal cells (ESC) incubated with NAC, AL, Br alone or in admixture.
Figure 4. TNFα expression by Endometriotic Endothelial cells (EEC) after stimulation with IL-1β.
Figure 5. Anti- proliferative effect of NAC, AL, BR alone or in admixture (MIX) on endometriotic endothelial cell (EEC).
Figure 6a. In vivo effect of Naxend®. The figure shows two SCID mice treated with Naxend® per os and two mice untreated (control).
Figure 6b. Graph showing the reduction of the number of the cysts in the mice treated with Naxend®.

### DESCRIPTION

Surprisingly, it has been found that a composition comprising N-acetyl cysteine, alpha-lipoic acid, and bromelain can be advantageously used in the treatment of endometriosis, dysmenorrhoea, and/or pelvic pain.

The term "treatment of endometriosis" according to the present invention means the reduction of the progression of the endometriotic mass and the reduction of the pain symptoms associated with the disease.

N-acetyl cysteine is a molecule which has the property of cleaving disulfide bonds, which explains why it is traditionally used as a mucolytic agent (it cleaves the disulfide bonds of mucoproteins) and as an antioxidant (for the regeneration of glutathione from GSSG to GSH). It has been highlighted that, since the state of inflammation in endometriosis is a chronic process, it also involves an increase in oxidative stress and up-regulation of the production of reactive oxygen species (ROS) which play not only a pro-inflammatory role but also the role of second messengers of cell proliferation and therefore a role in the evolution of endometriosis and the growth thereof in volumetric terms.

Since the use of N-acetyl cysteine in endometriosis can decrease the volume of the ectopic endometrium, it therefore allows the evolution of the disease to be stopped in much the same way as in conventional hormone treatment. However, unlike the aforesaid hormonal treatment, the strength of the N-acetyl cysteine is that it also impacts on the inflammation process in parallel, thereby simultaneously interrupting two mechanisms at their origins (More than antioxidant: N-acetyl-L-cysteine in a murine model of endometriosis. Pittaluga E, Costa G, Krasnowska E, Brunelli R, Lundeberg T, Porpora MG, Santucci D, Parasassi T. Fertil Steril. 2010 Dec;94(7):2905-8. doi: 10.1016/j.fertnstert.2010.06.038. Epub 2010 Jul 23. Reactive oxygen species controls endometriosis progression. Ngô C, Chereau C, Nicco C, Weill B, Chapron C, Batteux F. Am J Pathol. 2009 Jul;175(1):225-34. Inhibition of proliferation of endometrial stromal cells by trichostatin A, RU486, CDB-2914, N-acetylcysteine, and ICI 182780. Wu Y, Guo SW. Gynecol Obstet Invest. 2006;62(4):193-205. Effects of oxidants and antioxidants on proliferation of endometrial stromal cells. Foyouzi N, Berkkanoglu M, Arici A, Kwintkiewicz J, Izquierdo D, Duleba AJ. Fertil Steril. 2004 Oct; 82 Suppl 3:1019-22).

Alpha-lipoic acid (also known as thioctic acid) is a molecule composed of two disulfide groups which participate in redox reactions in the form of glutathione and ascorbic acid antioxidant agents. The importance of its antioxidant activity is bivalent: directly, it is an acceptor of radicals and indirectly, it activates other antioxidants, such as divalent ions (Fe²⁺, Mg²⁺, Cu²⁺). Therefore, by reducing the levels of oxidative stress, this compound contributes, acting synergistically with the N-acetyl cysteine to reduce the proliferation of endometriotic cells (Alfa-lipoic acid controls tumor growth and modulates hepatic redox state in Ehrlich-ascites-carcinoma-bearing mice. Al Abdan M.Scientific World Journal. 2012;2012:509838. Epub 2012 May 1. Antioxidant therapy: current status and future prospects. Firuzi O, Miri R, Tavakkoli M, Saso L. Curr Med Chem. 2011;18(25):3871-88. Review).

As far as bromelain is concerned, this is a molecule which expresses proteolytic activity and is extracted from the fruit *Ananas comosus.* Already commonly known in folk medicine, this compound has anti-inflammatory and anti-edematous properties. Numerous studies have demonstrated a strong anti-inflammatory action of bromelain through selective inhibition of COX-2 and, in particular, *in vivo* data has demonstrated decreased production of PGE2 after taking bromelain (Treatment with oral bromelain decreases colonic inflammation in the IL-10-deficient murine model of inflammatory bowel disease. Hale LP, Greer PK, Trinh CT, Gottfried MR. Clin Immunol. 2005 Aug; 116(2):135-42. In vivo and in vitro effects of bromelain on PGE(2) and SP concentrations in the inflammatory exudate in rats. Gaspani L, Limiroli E, Ferrario P, Bianchi M. Pharmacology. 2002 May; 65(2): 83-6).

Surprisingly, it has now been found that N-acetyl cysteine, alpha-lipoic acid, and bromelain, acting synergistically, impact on two specific aspects relating to the evolution of the pathology, namely the increase of the volume of the endometrium and the pain symptoms.

Furthermore, as clearly demonstrated by the pre-clinical data reported in the experimental section, the composition comprising N-acetyl cysteine, alpha-lipoic acid and bromelain have a surprising synergistic effect on the endometriotic endothelial cells reducing the inflammatory process and the excess of cell proliferation, related to the pathogenesis of endometriosis. It is in fact well known that endometriosis is defined as the presence of endometrial-like tissue outside the uterus, which induces a chronic, inflammatory reaction associated with pelvic pain and infertility. Vascular endothelium is known to play a critical role in regulation of inflammatory processes (Mantovani et al., Cytokines, 1992). A number of mediators, including cell adhesion molecules such as vascular cell adhesion molecule-1 (VCAM-1), pro-inflammatory cytokines, such as tumour necrosis factor-a (TNF-α), interleukin-6 (IL-6) and interleukin-8 (IL-8), and chemokines, such as monocyte chemotactic protein-1 (MCP-1), play key roles in the pathogenesis of endometriosis (Burney et al., 2012).

In particular, the mixture of N-acetyl cysteine, alpha-lipoic acid and bromelain proved to be more effective in inducing a down-regulation of VCAM expression , blocking the growth of endometriotic endothelial cells than the single drugs; and leading to an expression of TNFα lower then that of the single drugs.

The present invention therefore relates to a composition containing N-acetyl cysteine, alpha-lipoic acid, bromelain and, preferably, at least one physiologically acceptable excipient.

For the purpose of the present invention, the expression "consists essentially of' means that the claimed composition contains N-acetyl cysteine, alpha-lipoic acid and bromelain as the only active ingredients, optionally in combination with at least one physiologically acceptable excipient; that is, the percentage sum of N-acetyl cysteine, alpha-lipoic acid, bromelain and of the optional at least one physiologically acceptable excipient is 100.

According to one embodiment the composition of the present invention contains N-acetyl cysteine, alpha-lipoic acid and bromelain, wherein the N-acetyl cysteine is present in the said composition in an amount by weight ranging from 35% to 70%, preferably from 40% to 60%, and more preferably from 44% to 52%, with respect to the total weight of the composition.

Alpha-lipoic acid is present in the composition of the present invention in an amount by weight ranging from 10% to 25%, preferably from 12% to 20%, more preferably from 14 to 17%, with respect to the total weight of the composition.

Bromelain is present in the composition of the present invention in an amount by weight ranging from 0.1% to 10%, preferably from 1% to 5%, more preferably from 1.5% to 2%, with respect to the total weight of the composition.

According to the present invention, the aforesaid composition may be formulated in oral form, preferably in the form of a tablet or capsule, more preferably in the form of a coated tablet or capsule; the preferred coating according to the present invention is a gastro-resistant coating.

According to one aspect of the present invention, the composition has a delayed release and includes a core containing N-acetyl cysteine, alpha-lipoic acid, bromelain, at least one physiologically acceptable excipient, and a gastro-resistant coating.

According to a particularly preferred embodiment, the said delayed-release composition comprises:
a) a core consisting of: a controlled-release matrix in which alpha-lipoic acid is dispersed; N-acetyl cysteine; bromelain; at least one physiologically acceptable excipient;
b) a gastro-resistant coating.

According to the present invention, the term "physiologically acceptable excipient" means a substance devoid of any pharmacological effect of its own and which does not produce adverse reactions when administered to a mammal, preferably a human.

According to the present invention, the term "delayed-release formulation or composition" means a form of administration that allows the active ingredient contained therein to be released after a predetermined time.

According to the present invention, the term "matrix" means a matrix of a polymeric nature in which the active ingredient is dispersed in a homogenous manner.

According to the present invention, the term "controlled-release matrix" means a matrix that releases the active ingredient dispersed therein slowly over time and allows blood or tissue levels to be kept constant over a given period of time.

According to the present invention, the term "gastro-resistant coating" means a coating that allows the composition to cross the gastric tract without being damaged and then release the active ingredient or ingredients within the intestine.

In particular, the controlled-release matrix in which the alpha-lipoic acid is dispersed is composed of at least one polymer chosen from ethyl cellulose, methyl cellulose, and hydropropylmethyl cellulose. Said polymer is preferably ethyl cellulose.

The excipients usable are preferably chosen from microcrystalline cellulose, crosslinked carboxymethyl cellulose, lactose, corn starch, silicon dioxide, magnesium stearate, tribasic calcium phosphate, talc and/or polyvinylpyrrolidone; said excipients are preferably microcrystalline cellulose, silicon dioxide, talc, and magnesium stearate.

Preferably, excipients usable for the purposes of the present invention, including the polymers of the matrix, are present in an amount by weight ranging from 15% to 50%, more preferably from 20% to 40%, still more preferably from 25 to 35%, with respect to the total weight of the composition.

Suitable gastro-resistant coatings according to the present invention may be chosen from polymers and copolymers of acrylic and methacrylic acid, polyvinyl acetate phthalate, modified starch, glycerol, talc, or mixtures thereof. The gastro-resistant coating may also include one or more dyes.

The gastro-resistant coating according to the present invention is present in said composition in an amount by weight ranging from 5% to 20%, preferably from 7% to 15%, with respect to the total weight of the composition.

The gastro-resistant coating prevents the gastric absorption of the active ingredients, which involves a substantial first-pass hepatic metabolism effect and thus a poor bioavailability of said ingredients. Moreover, this coating preserves the active ingredients against rapid oxidation, in addition to masking the bad odour released from the N-acetyl cysteine.

The matrix in which, according to one aspect of the invention, the alpha-lipoic acid is dispersed allows the controlled release thereof (about 20-25% every two hours over a total of eight hours). Said controlled release allows to administer the composition of the present invention with a lower number of daily administrations, despite the very short half-life (15-20 minutes) of alpha-lipoic acid.

Such a mode of administration therefore involves fewer side effects and the composition of the present invention results more bioavailable with respect to conventional treatments.

A further aim of the present invention is the use of the aforesaid composition in the treatment of endometriosis, dysmenorrhoea, and/or pelvic pain, the recipient of the treatment preferably being a mammal, preferably a human, and even more preferably female.

According to one embodiment of the present invention, said composition is administered from once to four times a day, preferably twice a day.

The compositions of the present invention can be prepared with conventional methods well known to persons skilled in the art.

The following examples are given for illustrative and non-limiting purpose of the present invention.

### EXPERIMENTAL SECTION

### Example 1 (Tablet)

| | |
|---|---|
| Matris® Thioctic (equivalent to 200 mg of alpha-lipoic acid)* | 285 mg |
| N-acetyl cysteine | 600 mg |
| Bromelain 2500 GDU | 25 mg |
| Microcrystalline cellulose | 235 mg |
| Silicon dioxide | 10 mg |
| Vegetable magnesium stearate | 10 mg |
| Talc | 10 mg |

The tablet is prepared by mixing and subsequent compressing the components.

This is a matrix containing alpha-lipoic acid, ethyl cellulose, talc, and silicon dioxide, marketed by IPS International Products & Services S.r.1.

### CLINICAL DATA

### Example 2

A first group of five women with stage I-II endometriosis who had already undergone surgical treatment, wanted children and stopped chronic treatment with NSAIDs (non-steroidal anti-inflammatory drugs), but suffering from persistent, chronic pelvic pain were treated with the tablet from Example 1, three times a day for three 30-day cycles without interruption, following conventional treatment (NSAIDs) in the month preceding the start of administration of the product.

A second group of five women with a history of primary dysmenorrhoea, who wanted children and had refused to take NSAIDs were treated with the composition from Example 1, three times a day for five days per month during the perimenstrual period.

### FOLLOW-UP and OBSERVATIONAL RESULTS

All the women reported a significant reduction in pain symptoms (meaning thereby the level of abdominal-pelvic pain), as well as being able to carry out and resume normal work activity, and an enhancement of regular sexual activity which had previously affected by deep dyspareunia.

Non-observational data based on more larger, homogeneous collection of randomised case studies, selected based on the genesis of chronic pelvic pain (primary dysmenorrhoea, endometriosis, premenstrual syndrome, etc.) will be able to confirm these observational findings and above all validate the possible effectiveness of the product.

### PRE-CLINICAL DATA

### Example 3. Evaluation of the anti-inflammatory effect

### Analysis of the expression by human endometriotic endothelial cells (EECs), stimulated with TNFα, of VCAM-1 by ELISA.

We tested 3 different population of endothelial cells isolated from ovaric cysts obtained from 3 different patients.

The cells were growth to the confluence in 96 well plates and incubated with drugs at the following concentrations: N-acetyl cysteine (NAC) 5µg/ml, alpha-lipoic acid (AL) 10µg/ml, bromelain (Br) 15µg/ml, alone or in admixture (MIX), overnight at 37°C 5% CO₂. Successively the cells were stimulated overnight with TNFα (100ng/ml), washed with Dulbecco's PBS added with 2% BSA (Bovine Serum Albumine, fractionV, Sigma-Aldrich) and CaCl₂-MgCl₂ 0,7mM (Sigma-Aldrich) and then incubated with mouse mAb anti human VCAM-1 (Sigma-Aldrich) 5µg/ml for 90 minutes at room temperature. The binding of primary antibody was reveled incubating the cells with a polyclonal anti-mouse IgG conjugated with alkaline phosphatase. The enzymatic reaction was developed with PNPP (p-nitrophenyl phosphate) (Sigma-Aldrich; 1 mg/ml) as substrate and read kinetically at 405 nm using a Titertek Multiskan ELISA reader (Flow Labs, Milano, Italy).

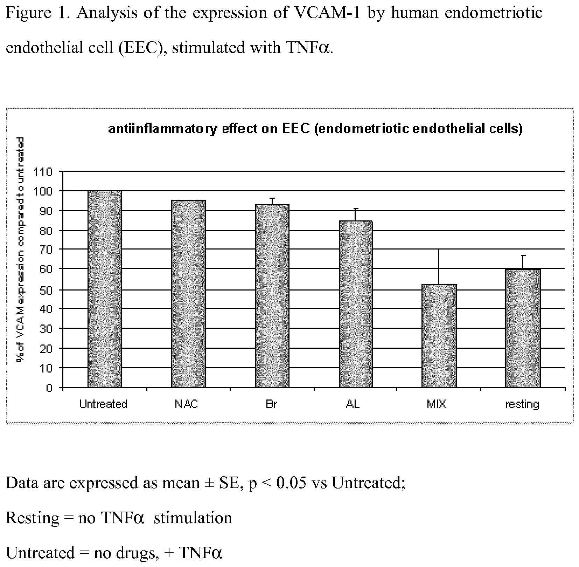

As showed in Figure 1, Naxend® (MIX) is able to induce a down-regulation of VCAM expression, leading to lower level of VCAM expression than the unstimulated EEC (resting).

### Evaluation of mRNA expression by EEC or ESC of inflammatory cytokines.

Quantitative PCR (qPCR) of mRNA extracted from a confluent monolayer of EEC (or ESC) incubated with drugs (concentration of: NAC 5µg/ml, AL 10µg/ml, Br 15µg/ml), alone or in admixure, for 48h at 37° C 5% CO2. Successivly the cells were stimulated overnight with IL-1β (10U/ml). qPCR was carried out on Rotor-Gene 6000TM (Corbett, Explera, Ancona, Italy) using iQ TM SYBR Green Supermix (Bio-Rad, Milan, Italy). The melting curve was recorded between 55°C and 99°C with a hold every 2 sec. The relative amount of the gene of interest in each sample was normalized with 18s and expressed as arbitrary units (AU) considering 1AU that obtained in EEC (or ESC) resting used as positive control. We evaluated the modulation of the expression of inflammatory cytokines, such as TNFα, IL-8 and IL-6. The sequences and the melting temperature of the primers, used for qPCR analysis, are reported in Table 1.

**Table 1.**

| GENE | SEQUENCE | TM | G.I. |
|---|---|---|---|
| 18S | For ATCCCTGAAAAGTTCCAGCA | 61O | NM_022551 |
| | Rev CCCTCTTGGTGAGGTCAATG | | |
| | CATTGACCTCACCAAGAGGG | | |
| IL6 | For GTACATCCTCGACGGCATC | 60o | NM_000600.3 |
| | Rev CCAGGCAAGTCTCCTCATTG | | |
| | CAATGAGGAGACTTGCCTGG | | |
| IL8 | For AGGTGCAGTAGTTTTGCCAAGGA | 60O | NM_000584 |
| | Rev TTTCTGTGTTGGCGCAGTGT ACACTGCGCCAACACAGAAA | | |
| TNF α | For GGCCCAGGCAGTCAGAT | 66O | NM_000594.3 |
| | Rev GGGGCTCTTGATGGCAGAGA | | |

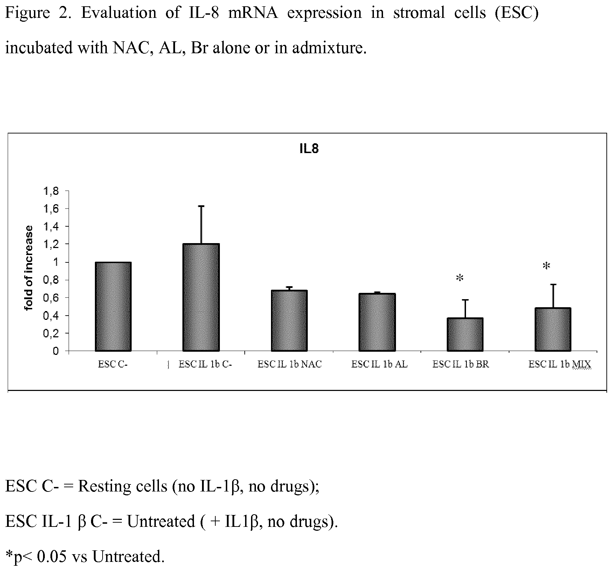

As shown in Figure 2 the up-regulation induced in ESC by IL-1β of IL-8 expression was blocked by the treatment with all the individual drugs, mainly with bromelin, and this effect was maintained with the mixture.

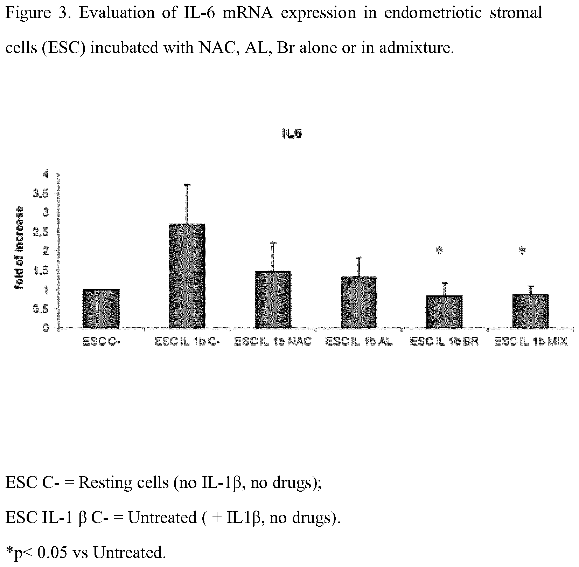

In Figure 3 it is shown that the up-regulation induced in ESC by IL-1β of IL-6 expression was blocked by the treatment with all the individual drugs, and that the effect of the mixture is similar to that of bromelin.

The results obtained show that the anti-inflammatory effect of the mixture is higher on TNFα expression than on IL-8 and IL-6 expression.

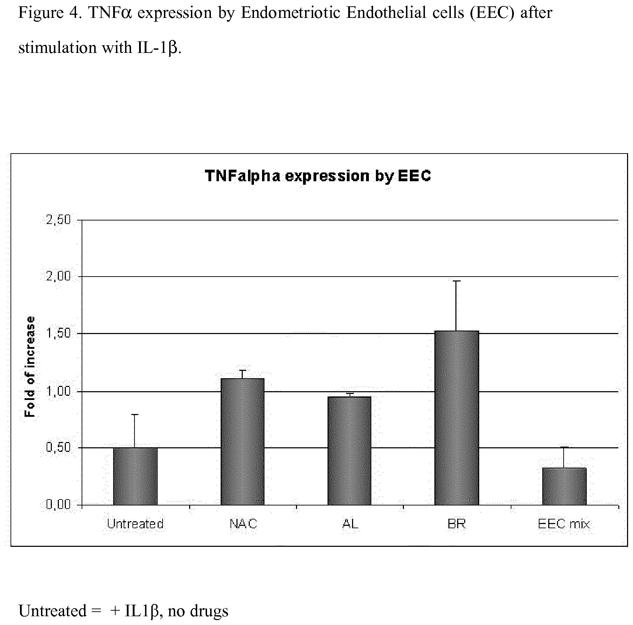

Figure 4 shows the TNFα expression by Endometriotic Endothelial cells (EEC) after stimulation with IL-1β. In the experimental conditions used, the results show that individual drugs induced an upregulation of the basal expression of TNFα by EEC. Interestingly, the treatment of the cell with three drugs together led to an expression of TNFα lower then that of the single drugs and of the untreated cells.

### Anti proliferative effect on EEC

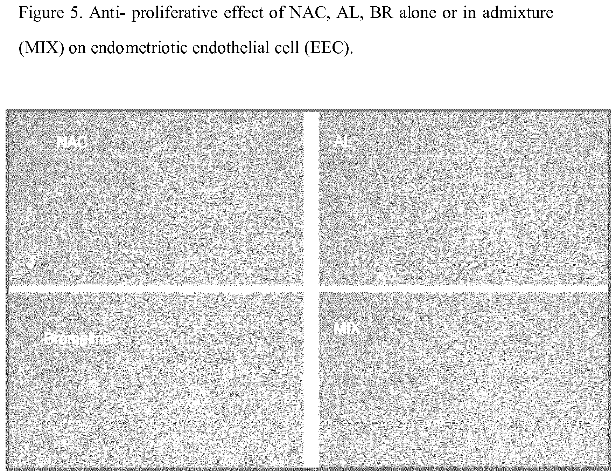

EEC (6x10⁴) were plated on 8 chamber culture slide, precoated with human fibronectin in medium serum free (Gibco) and stimulated for 72h with drugs (concentration of: NAC 10µg/ml, AL 20µg/ml, Br 3µg/ml), alone or in association, at 37°C 5% CO₂. Figure 5 shows that an admixture of NAC, AL and Br (MIX) is capable to block the growth of cells (only small areas of the figure show the presence of cells), while the single drug alone does not show a reduction of cell proliferation. The images were catch with an inverted Light microscope (magnification 20X) and the pictures acquired using a Canon Power Shot A640 photocamera.

### In vivo effect of Naxend®

Development of the *in vivo* model.

Endometriotic tissue from three peritoneal cysts was collected in sterile PBS and then suspended as coarse fragments. Endometriotic tissue was then loaded in 3ml syringes, and standardized with respect to volume and weight. A volume of 0.5ml of human cyst, i.e. the equivalent of 0.4g of wet tissue, was injected using a 16-g needle into the peritoneal cavity of SCID mice. Hormonal therapy with 17-beta-estradiol-3-benzoate (Sigma-Aldrich, 30 micrograms/kg i.m.) was initiated at the time of injection and at 3 day intervals thereafter.

**Implanting Tissue.** Twenty-one days following injection the animals were killed and implanted endometriotic lesion were identified, resected and collected in formalin 10%. Four SCID mice were injected with endometriotic tissue. Two SCID mice were treated with Naxend® per os and two mice untreated were used as control.

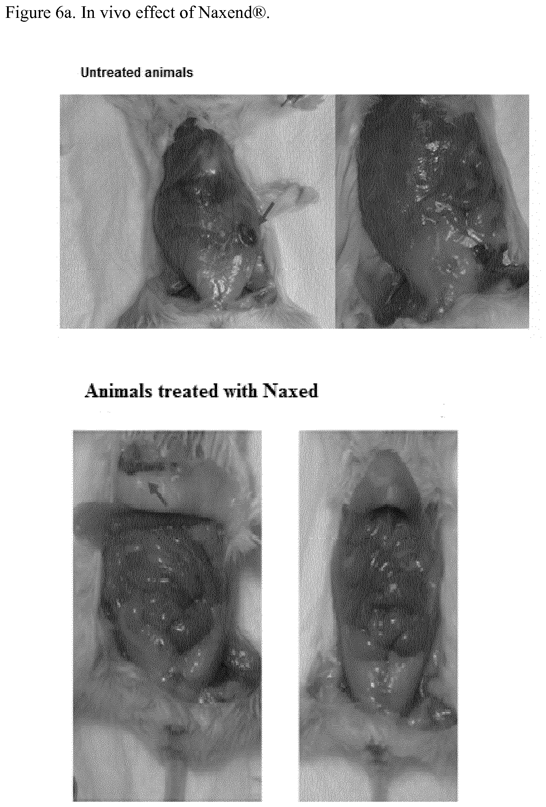

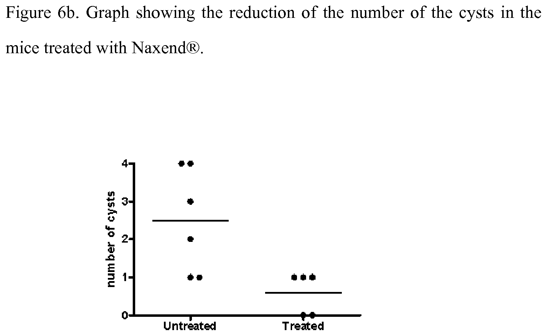

Figure 6a and the graph in Figure 6b show that that the mice treated with Naxend® have a reduction of the number of the cysts.

A new mouse model was set up, modifying the animal model described by Awwad and colleagues (Human Reprod. 1999) and by Guemmer and colleagues (Human Reprod. 2001). In particular human endometriotic tissue was used instead of normal human endometrium. This method improve the quality of the data since the effect of the drugs are on the real endometriotic cells. The difficulties of this new model were the limited availability of the tissue and the variability of the patients.

The dose was chosen on the bases of several works that used the NAC treatment on mice (Ivanovski et al., Kidney Int. 2005; Onalan et al., Arch Gynecol Obstet. 2014; Pittaluga et al., Fertility and Sterility 2010) and the relative proportions of the other drugs (AL and Br) were calculated on the bases of the NAXEND® composition.

The same administration route of the drugs was used for patients (p.o.) and this also affects the variability of the results. Despite this a statistical significance was achieved in terms of the number of cysts formed in the peritoneum.

## Claims

1. A composition containing N-acetyl cysteine, alpha-lipoic acid and bromelain, **characterised in that** N-acetyl cysteine is in an amount by weight ranging from 35% to 70%, preferably from 40% to 60%, with respect to the total weight of the composition.

2. A composition according to claim 1, **characterised in that** N-acetyl cysteine is in an amount by weight ranging from 44% to 52%, with respect to the total weight of the composition.

3. A composition according to claim 1, **characterised in that** alpha-lipoic acid is in an amount by weight ranging from 10% to 25%, preferably from 12% to 20%, with respect to the total weight of the composition.

4. A composition according to claim 3, **characterised in that** alpha-lipoic acid is in an amount by weight ranging from 14% to 17%, with respect to the total weight of the composition.

5. A composition according to claim 1, **characterised in that** bromelain is in an amount by weight ranging from 0.1% to 10%, preferably from 1% to 5%, with respect to the total weight of the composition.

6. A composition according to claim 5, **characterised in that** bromelain is in an amount by weight ranging from 1.5% to 2%, with respect to the total weight of the composition.

7. A composition according to any one of the previous claims, **characterised in that** said composition is formulated in an oral form, preferably in the form of a tablet or capsule.

8. A composition according to any one of the previous claims, **characterised in that** said composition is gastro-resistant coated.

9. A composition according to claim 8, **characterised in that** said gastro-resistant coating is present in an amount ranging from 5% to 20%, preferably from 7% to 15%, with respect to the total weight of the composition.

10. A composition, according any of the previous claims, **characterised in that** said composition comprises at least one physiologically acceptable excipient.

11. A composition according to claim 10, **characterised in that** said at least one excipient is present in an amount ranging from 15% to 50%, more preferably from 20% to 40%, and still more preferably from 25 to 35%, with respect to the total weight of the composition

12. A composition according to any one of the previous claims, for use in the treatment of endometriosis, dysmenorrhoea and/or pelvic pain.

13. A composition for use according to claim 12, **characterised in that** the recipient of the treatment is a mammal, preferably a human, and still more preferably female.

14. A composition for use according to claim 12, **characterised in that** said composition is administered once to four times a day, preferably twice a day.

## Patentansprüche

1. Zusammensetzung, welche N-Acetylcystein, alpha-Lipoinsäure und Bromelain umfasst, **dadurch gekennzeichnet, dass** N-Acetylcystein in einer Gewichtsmenge im Bereich von 35% bis 70% vorhanden ist, vorzugsweise von 40% bis 60%, in Bezug auf das Gesamtgewicht der Zusammensetzung.

2. Zusammensetzung nach Anspruch 1, **dadurch gekennzeichnet, dass** N-Acetylcystein in einer Gewichtsmenge im Bereich von 44% bis 52% vorhanden ist, bezogen auf das Gesamtgewicht der Zusammensetzung.

3. Zusammensetzung nach Anspruch 1, **dadurch gekennzeichnet, dass** alpha-Liponsäure in einer Gewichtsmenge im Bereich von 10% bis 25%, vorzugsweise von 12% bis 20%, bezogen auf das Gesamtgewicht der Zusammensetzung.

4. Zusammensetzung nach Anspruch 3, **dadurch gekennzeichnet, dass** alpha-Liponsäure in einer Gewichtsmenge im Bereich von 14% bis 17% vorhanden ist, bezogen auf das Gesamtgewicht der Zusammensetzung.

5. Zusammensetzung nach Anspruch 1, **dadurch gekennzeichnet, dass** Bromelain in einer Gewichtsmenge im Bereich von 0,1 % bis 10%, vorzugsweise von 1 % bis 5% vorhanden ist, bezogen auf das Gesamtgewicht der Zusammensetzung.

6. Zusammensetzung nach Anspruch 5, **dadurch gekennzeichnet, dass** Bromelain in einer Gewichtsmenge im Bereich von 1,5% bis 2% vorhanden ist bezogen auf das Gesamtgewicht der Zusammensetzung.

7. Zusammensetzung nach einem der vorstehenden Ansprüche, **dadurch gekennzeichnet, dass** die Zusammensetzung in einer oralen Form formuliert ist, vorzugsweise in Form einer Tablette oder Kapsel.

8. Zusammensetzung nach einem der vorstehenden Ansprüche, **dadurch gekennzeichnet, dass** die Zusammensetzung Magensaft-resistent beschichtet ist.

9. Zusammensetzung nach Anspruch 8, **dadurch gekennzeichnet, dass** die Magensaftresistente Beschichtung in einer Menge im Bereich von 5% bis 20%, vorzugsweise von 7% bis 15% vorhanden ist, bezogen auf das Gesamtgewicht der Zusammensetzung.

10. Zusammensetzung nach einem der vorstehenden Ansprüche, **dadurch gekennzeichnet, dass** die Zusammensetzung mindestens einen physiologisch annehmbaren Exzipienten umfasst.

11. Zusammensetzung nach Anspruch 10, **dadurch gekennzeichnet, dass** der mindestens eine Exzipient in einer Menge vorhanden ist im Bereich von 15% bis 50%, mehr bevorzugt von 20% bis 40%, und noch mehr bevorzugt 25 bis 35%, bezogen auf das Gesamtgewicht der Zusammensetzung

12. Zusammensetzung nach einem der vorstehenden Ansprüche zur Verwendung bei der Behandlung von Endometriose, Dysmenorrhoe und/oder Beckenschmerzen.

13. Zusammensetzung zur Verwendung nach Anspruch 12, **dadurch gekennzeichnet, dass** der Empfänger der Behandlung ein Säugetier ist, vorzugsweise ein Mensch, und noch mehr bevorzugt eine Frau.

14. Zusammensetzung zur Verwendung nach Anspruch 12, **dadurch gekennzeichnet, dass** die Zusammensetzung einmal oder viermal pro Tag verabreicht wird, vorzugsweise zweimal am Tag.

## Revendications

1. Composition contenant de la N-acétyl cystéine, de l'acide alpha-lipoïque et de la bromélaïne, **caractérisée en ce que** la N-acétyl cystéine est en une quantité en poids se situant dans la plage de 35 % à 70 %, de préférence de 40 % à 60 %, par rapport au poids total de la composition.

2. Composition selon la revendication 1, **caractérisée en ce que** la N-acétyl cystéine est en une quantité en poids se situant dans la plage de 44 % à 52 %, par rapport au poids total de la composition.

3. Composition selon la revendication 1, **caractérisée en ce que** l'acide alpha-lipoïque est en une quantité en poids se situant dans la plage de 10 % à 25 %, de préférence de 12 % à 20 %, par rapport au poids total de la composition.

4. Composition selon la revendication 3, **caractérisée en ce que** l'acide alpha-lipoïque est en une quantité en poids se situant dans la plage de 14 % à 17 %, par rapport au poids total de la composition.

5. Composition selon la revendication 1, **caractérisée en ce que** la bromélaïne est en une quantité en poids se situant dans la plage de 0,1 % à 10 %, de préférence de 1 % à 5 %, par rapport au poids total de la composition.

6. Composition selon la revendication 5, **caractérisée en ce que** la bromélaïne est en une quantité en poids se situant dans la plage de 1,5 % à 2 %, par rapport au poids total de la composition.

7. Composition selon l'une quelconque des revendications précédentes, **caractérisée en ce que** ladite composition est préparée sous une forme orale, de préférence sous la forme d'un comprimé ou d'une capsule.

8. Composition selon l'une quelconque des revendications précédentes, **caractérisée en ce que** ladite composition présente un revêtement gastro-résistant.

9. Composition selon la revendication 8, **caractérisée en ce que** ledit revêtement gastro-résistant est présent en une quantité se situant dans la plage de 5 % à 20 %, de préférence de 7 % à 15 %, par rapport au poids total de la composition.

10. Composition selon l'une quelconque des revendications précédentes, **caractérisée en ce que** ladite composition comprend au moins un excipient physiologiquement acceptable.

11. Composition selon la revendication 10, **caractérisée en ce que** ledit au moins un excipient est présent en une quantité se situant dans la plage de 15 % à 50 %, plus préférablement de 20 % à 40 %, et encore plus préférablement de 25 à 35 %, par rapport au poids total de la composition.

12. Composition selon l'une quelconque des revendications précédentes, pour utilisation dans le traitement de l'endométriose, de la dysménorrhée et/ou des douleurs pelviennes.

13. Composition pour utilisation selon la revendication 12, **caractérisée en ce que** le destinataire du traitement est un mammifère, de préférence un humain, et encore plus préférablement une femme.

14. Composition pour utilisation selon la revendication 12, **caractérisée en ce que** ladite composition est administrée une à quatre fois par jour, de préférence deux fois par jour.
